# EUROPEAN PATENT SPECIFICATION

(11) **EP 1 926 424 B1**
(45) Date of publication and mention of the grant of the patent: **19.08.2009**
(21) Application number: 06795797.7
(22) Date of filing: 28.08.2006
(51) Int. Cl.: A61B 5/053, G01N 27/02, G01N 27/90

(54) **SYSTEM AND METHOD FOR INDUCTIVELY MEASURING THE BIO-IMPEDANCE OF A CONDUCTIVE TISSUE**
SYSTEM UND VERFAHREN ZUM INDUKTIVEN MESSEN DER BIOIMPEDANZ EINES LEITFÄHIGEN GEWEBES
SYSTEME ET PROCEDE PERMETTANT DE MESURER PAR COUPLAGE INDUCTIF LA BIO-IMPEDANCE D'UN TISSU CONDUCTEUR

(30) Priority: 07.09.2005 EP 05108176
(43) Date of publication of application: 04.06.2008
(73) Proprietor: Philips Intellectual Property & Standards GmbH, 20099 Hamburg (DE); Koninklijke Philips Electronics N.V., 5621 BA Eindhoven (NL)
(72) Inventor: IGNEY, Claudia Hannelore, 52066 Aachen (DE); WAFFENSCHMIDT, Eberhard, 52066 Aachen (DE); BRAUERS, Andreas, 52066 Aachen (DE); TE VRUGT, Juergen, 52066 Aachen (DE)
(74) Representative: Schouten, Marcus Maria
(86) International application number: PCT/IB2006/052979
(87) International publication number: WO 2007/029138

(56) References cited:
- WO-A2-20/05031282
- US-A- 3 249 869
- US-A1- 2004 075 429

## Description

The present invention relates to a system and method for inductively measuring the bio-impedance of a conductive tissue. Furthermore the invention relates to a computer program for operating such a system.

The inductive measurement of bio-impedances is a known method to determine various vital parameters of a human body in a non-contact way. The operating principle is the following: Using a generator coil, an alternating magnetic field is induced in a part of the human body. This alternating magnetic field causes eddy currents in the tissue of the body. Depending on the type and conductivity of the tissue, the eddy currents are stronger or weaker. The eddy currents cause a secondary magnetic field, which can be measured as an induced voltage in a sensor coil.

The inductive measurement of the bio-impedance has been shown to allow the non-contact determination of several parameters, e.g. breath action and depth, heart rate and change of the heart volume and blood glucose level, as well as fat or water content of the tissue.

In order to enhance the sensitivity and the signal to noise ratio (SNR) of such measuring systems, different coil arrangements have been proposed for magnetic field compensation. However, in practice a large effort is being devoted to manually adjust the coils (e.g. using adjustable mounting screws) to reach an optimal setup. Furthermore said adjustment has to be repeated if the sensor system is moved or changed.

US 3,249,869 describes an apparatus for measuring the electrical properties of a conductive moving fluid that comprises an arrangement of three coils, US 2004/075429 describes an eddy current testing probe and WO 2005/031282 describes a system and method for measuring volumes and areas using electromagnetic induction techniques.

It is an object of the present invention to provide a fast, simple and reliable adjustment technique for an inductively bio-impedance measuring system with separate generator and sensor coils.

This object is achieved according to the invention by a system for inductively measuring the bio-impedance of a conductive tissue, the system comprising a generator coil adapted for generating a primary magnetic field, said primary magnetic field inducing an eddy current in the tissue, a separate sensor coil adapted for sensing a secondary magnetic field, said secondary magnetic field being generated as a result of said eddy current, with the axis of the sensor coil being orientated substantially perpendicular to the flux lines of the primary magnetic field, and a number of shimming coil adapted for generating a tertiary magnetic field in a way that in the sensor coil the primary magnetic field is cancelled out.

The object of the present invention is also achieved by a method for inductively measuring the bio-impedance of a conductive tissue, the method comprising the steps of: arranging a generator coil and a separate sensor coil, with the axis of the sensor coil being orientated substantially perpendicular to the flux lines of a primary magnetic field generated by means of the generator coil, said primary magnetic field inducing an eddy current in the conductive tissue, sensing a secondary magnetic field by means of the sensor coil, said secondary magnetic field being generated as a result of said eddy current, and generating a tertiary magnetic field by means of a shimming coil in a way that in the sensor coil the primary magnetic field is cancelled out.

The object of the present invention is also achieved by a computer program for operating a system for inductively measuring the bio-impedance of a conductive tissue, the system comprising a generator coil adapted for generating a primary magnetic field, said primary magnetic field inducing an eddy current in the tissue, a sensor coil adapted for sensing a secondary magnetic field, said secondary magnetic field being generated as a result of said eddy current, with the axis of the sensor coil being orientated substantially perpendicular to the flux lines of the primary magnetic field, and a shimming coil, the program comprising computer instructions to automatically control the shimming coil to generate a tertiary magnetic field in a way that in the sensor coil the primary magnetic field is cancelled out. The technical effects necessary according to the invention can thus be realized on the basis of the instructions of the computer program in accordance with the invention. Such a computer program can be stored on a carrier such as a CD-ROM or it can be available over the Internet or another computer network. Prior to being executed, the computer program is loaded into the computer by reading the computer program from the carrier, for example by means of a CD-ROM player, or from the Internet, and storing it in the memory of the computer. The computer includes inter alia a central processor unit (CPU), a bus system, memory means, e.g. RAM or ROM etc., storage means, e.g. floppy disk or hard disk units etc. and input/output units. Alternatively the inventive method could be implemented in hardware, e. g. using one or more integrated circuits.

A core idea of the invention is to complete the mechanical adjustment of the sensor coil (as known from the prior art) with an additional electronic adjustment. Said electronic adjustment can be automated and performed in situ during operation of the system.

The invention suggests that the sensor coil is mechanically arranged in a way that the native (primary) magnetic field generated by the generator coil is cancelled out as far as possible in the sensor coil and only the (secondary) magnetic field generated by eddy currents in the conductive tissue is sensed. In other words, the generator coil and the sensor coil are arranged beforehand, preferably during the installation setup of the system, in a way that approximately no magnetic net flux from the generator coil passes through the sensor coil. In other words, inside the sensor coil the primary magnetic field lines are substantially (i.e. nearly) tangential to the sensor coil, i.e. inside the sensor coil the axis of the sensor coil is substantially perpendicular to the magnetic field lines of the primary magnetic field. During operation of the system, the conductive tissue creates a secondary magnetic flux through the sensor coil, which is not zero. However, the primary magnetic flux through the sensor coil will not be precisely zero because of the influence of temperature or modifications of the coil position etc. during operation of the system.

Now, according to the invention, the sensor coil is provided with a shimming coil for electronic adjustment. Into the shimming coil a defined current is injected. The amplitude of the current is adjusted such that the (tertiary) magnetic field generated by the shimming coil completely cancels the native (primary) magnetic field. Thus, the resulting magnetic flux (net flux) of the primary magnetic field through the sensor coil is made zero. Only the tertiary magnetic field originating from the eddy current in the conductive tissue is sensed by the sensor coil. This way the sensitivity of the sensor coil and the signal to noise ratio (SNR) of the sensing arrangement is improved significantly and the manufacturing effort is reduced to allow a low cost mass production. With the invention a fast, simple and reliable adjustment technique is provided. No extensive (manual) sensor adjustment is necessary.

The term "conductive tissue" has to be understood as conductive organic material, e.g. a body of a human or animal or, a plant. Furthermore the term "conductive tissue" comprises substances, like water, muscle, fat, blood or cerebrospinal fluid (CSF). "Conductive tissue" further comprises non-organic conducting or low conducting tissue of any kind, in particular for material testing.

The invention can be used with a contactless medical diagnostic system that measures inductively the bio-impedance of a user's body. Such a system allows an easy and comfortable diagnosis of vital parameters like the heart rate, tissue water content or blood glucose level to supervise a user without the need of applying any kind of devices to the user's body. This method can also be used to measure the position of the user, the breathing frequency, movement etc.

It will be evident to those skilled in the art that the invention is not limited to a system and method using just one generator coil, one sensor coil and one shimming coil. The invention can be realized using a larger number of sensor coils together with a corresponding number of shimming coils. Furthermore the invention can be realized using more than one generator coil.

In particular if a large number of measuring data is needed, a larger number of generator coils, sensor coils and shimming coils (e.g. 4, 8, 16 or 32 or any other kind of coil combinations) may be employed. The coils are then preferably arranged in form of an array or a matrix or any other way that the primary field is canceled out. Such a larger number of coils may be used e.g. in order to implement a magnetic induction tomography (MIT) system or a multi channel system for monitoring, e.g. because different parts of the user's lungs need to be monitored e.g. due to oedema development in the lungs.

These and other aspects of the invention will be further elaborated on the basis of the following embodiments which are defined in the dependent claims.

According to a preferred embodiment of the invention the shimming coil and the sensor coil are located in a way that the axis of the shimming coil is orientated parallel to the axis of the sensor coil. In this way the shimming coil allows to create a magnetic flux in direction of the primary magnetic field and so cancels out the resulting magnetic field.

According to another preferred embodiment of the invention the shimming coil is implemented as one or more auxiliary windings of the sensor coil. This way sensor coil and shimming coil can be integrated into a single component, which reduces manufacturing costs and time and effort for coil setup.

According to another preferred embodiment of the invention a control unit for controlling the shimming coil is connected to the shimming coil, said control unit being adapted for providing a shimming current to the shimming coil. Said control unit is preferably adapted for receiving a partial amount of the current of the generator coil in order to apply this current to the shimming coil. Because a fraction of the generator coil current is applied to the shimming coil, the field of the generator coil and the field of the shimming coil show a phase difference of 180°. Using the induced signal in the generator coil allows to cancel the resulting flux of the primary magnetic field locally in the sensor coil using an electronic way. In other words the current of the shimming coil can be adjusted electronically (and preferably automatically) using the control unit. Furthermore in this way the signal in the sensor coil can be (automatically) minimized if no tissue is near the measurement system.

According to another preferred embodiment of the invention, the control unit comprises a controllable potentiometer or a controllable resistor for adjusting the amplitude of the shimming current, thereby being controlled by the control unit. Thus, the electronic adjustment can be performed using a very simple setup.

According to another preferred embodiment of the invention the control units comprise a phase shifter module adapted for shifting the phase of the shimming current. Thus parasitic (e.g. capacitive) effects due to measuring conditions can be compensated in an easy way.

According to another preferred embodiment of the invention the shimming coil is considerably smaller than the generator coil and/or the shimming current applied to the shimming coil is very low compared to the generator coil current applied to the generator coil. By this means it can be assured, that there are no or only negligible eddy currents produced by the shimming coil. Such eddy currents would effect the measuring and impair the SNR of the measurement.

According to yet another preferred embodiment of the invention the sensor coil is a SMD (surface mounted device) coil attached to a printed circuit board by means of two attachment points and the shimming coil comprises a number of PCB (printed circuit board)-tracks and a corresponding number of wires, with said PCB-tracks being positioned between said two attachment points and beneath said SMD coil and said wires running across said SMD coil. With such an embodiment a cheap and small sensor unit can be achieved with an electronically adjustable sensor coil.

These and other aspects of the invention will be described in detail hereinafter, by way of example, with reference to the following embodiments and the accompanying drawings; in which:
Fig. 1 shows a schematic view of coils in coaxial alignment (prior art),
Fig. 2 shows a schematic view of coils without conductive tissue in a normal alignment (prior art),
Fig. 3 shows another schematic view of coils with conductive tissue in a normal alignment (prior art),
Fig. 4 shows a schematic view of a measuring system according to the invention,
Fig. 5 shows a schematic block diagram of a measuring system according to the invention,
Fig. 6 shows a schematic view of coils in a normal alignment according to the invention,
Fig. 7 shows a schematic view of coils in another alignment according to the invention, and
Fig. 8 shows a schematic view of an embodiment of the invention realized in SMD technique.

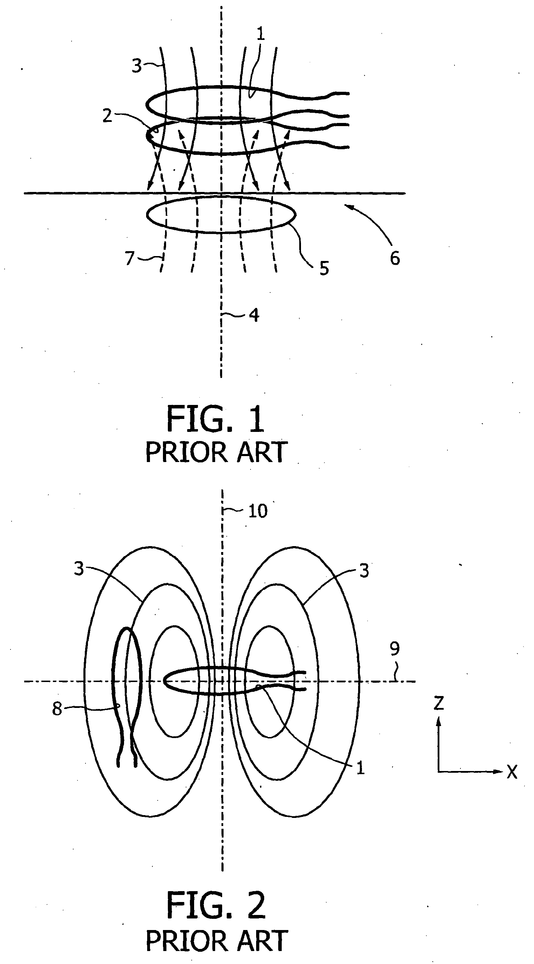

Fig. 1 illustrates the general principle of measuring eddy currents in a conductive tissue of a user's body using an axial alignment of a generator coil 1 and a sensor coil 2 as known from the prior art. An alternating current is fed into the generator coil 1 and produces an alternating primary magnetic field 3 (in all figures the flux lines are shown representing the magnetic field). The sensor coil 2, being axially aligned with the generator coil 1, senses the primary magnetic field 3. The axis 4 is shown as dot and dash line. If the primary alternating magnetic field 3 passes through a conducting material, e.g. tissue 6 of the user's body, eddy currents 5 are induced. The eddy currents 6 are illustrated schematically in form of a loop. These eddy currents 5 will also produce an alternating secondary magnetic field 7 (dotted lines). As a result the sensor coil 2 measures the primary and the secondary (i.e. a perturbed) field.

If such a coil arrangement is used in a magnetic induction tomography (MIT) system, reducing the effect of the primary magnetic field 3 will help to increase the sensitivity and the SNR (signal to noise ratio) of the measurement. The primary magnetic field 3 can approximately be cancelled using different specific sensor alignments. In one of those field compensation schemes a high sensitivity is achieved in that a sensor coil is intended to be arranged such that the primary magnetic field 3 does not induce a signal in the sensor coil. The aim of this approach is that only eddy currents 5 in the conductive tissue 6 may induce a signal.

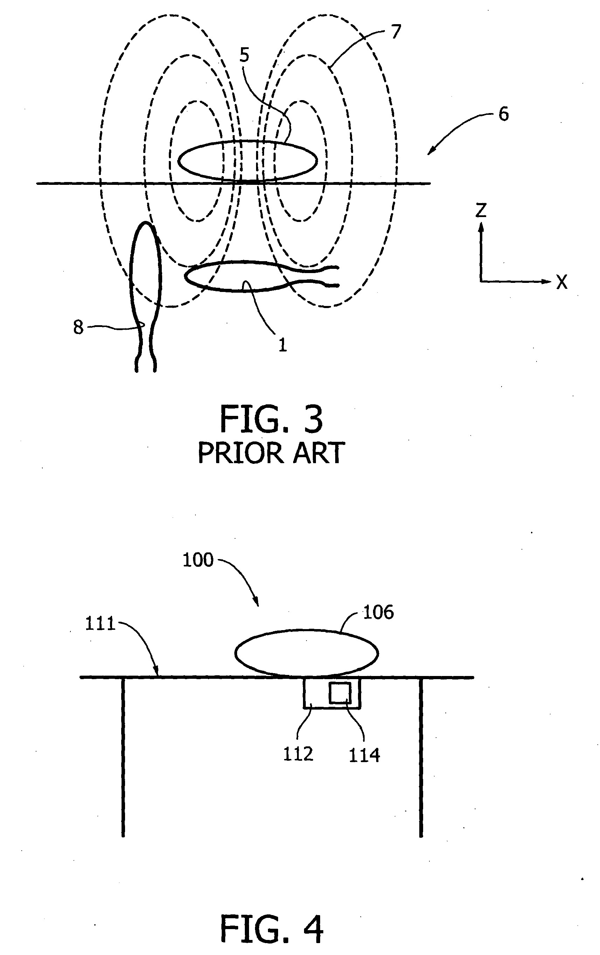

For multi-channel systems, the adoption of a planar array geometry in principle allows primary magnetic field compensation for all combinations of generator coil and sensor coil. In Fig. 2 (prior art) a normal (i.e. vertical) alignment is shown with no conductive tissue in measuring position as known from the prior art. The generator coil 1 and the sensor coil 8 are placed on a common plane (XZ-plane), with the axis 9 of the sensor coil 8 orientated perpendicular to the axis 10 of the generator coil 1. At the same time the axis 9 of the sensor coil 8 is orientated substantially perpendicular to the flux lines of the primary magnetic field 3, such that approximately no net flux from the generator coil 1 passes through the sensor coil 8. In other words, the sensor coil 8 is aligned with respect to the generator coil 1 in a way that the flux lines of the primary magnetic field 3 are substantially tangential (90°) to the axis 9 of the sensor coil 8. In Fig. 2 there is no medium in measuring position. In Fig. 3 (prior art) there is conducting tissue 6 of the body of a user in a measuring position. The conductive tissue 6 is of the plane and therefore creates a secondary magnetic field 7 and a secondary magnetic flux, which is not zero. The secondary magnetic flux can be sensed in the sensor coil 8. Such a sensor arrangement is also called Bₓ sensor or normal alignment, since the sensor coil 8 is adapted to measure a magnetic flux in X-direction. In Fig. 3 the flux lines of the primary magnetic field 3 are not shown for the reason of clarity. However, the primary and secondary magnetic fields 3, 7 are superposing each other.

From the prior art it is known to adjust the position of the coils 1, 8, in particular the position of the sensor coil 8, by means of adjustable plastic screws (not shown). In other words, in order to increase the SNR the signal of the primary magnetic field 3 has to be reduced by turning the screws. However, by this means a coil alignment can be reached, which is only substantially (i.e. nearly) perpendicular.

In Fig. 4 a simplified and schematic view of a measurement system 100 for inductively measuring the bio-impedance of a conducting tissue 106 within the body of a user according to the present invention is given. For example the system 100 may be a tomographic system adapted to use bio-impedance measurements in a tomographic way to obtain a conductivity distribution over time in a user's body in two or three dimensions or as a single image. In another example the system 100 may be a bed for monitoring a user by means of bio-impedance measurement. In another example the system 100 may be adapted for performing a single channel or multi channel measurement, e.g. for monitoring vital signs of a user. From measurements over time, certain characteristics, like volume changes of the heart or lungs of the user can be calculated. In the following description analyzing units or other components of the system 100 which are employed for evaluating the measurement results are not described further. However, in order to obtain feasible results, such analyzing components have to be used together with the described measuring components.

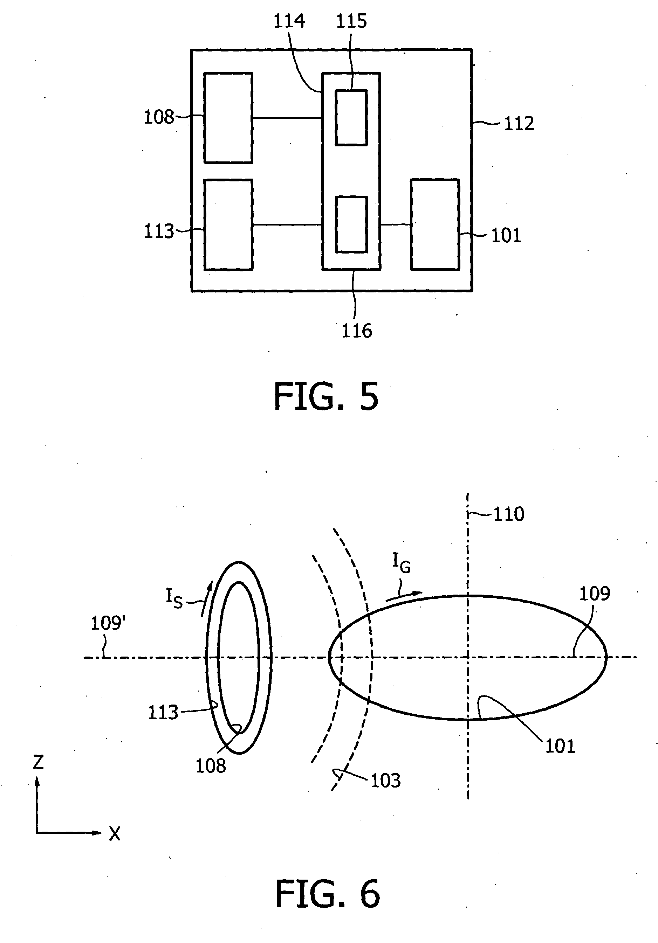

The conductive tissue 106 is positioned on a support 111, e.g. a bed or a measuring desk. Near the tissue 106 the measuring unit 112 is positioned, said measuring unit 112 comprising a generator coil 101 adapted for generating a primary magnetic field 103 (flux lines are shown as dotted lines), a separate sensor coil 108 adapted for sensing a secondary magnetic field, a shimming coil 113 adapted for generating a tertiary magnetic field and a control unit 114 adapted for controlling the shimming coil 113, said control unit 114 being connected to the shimming coil 113, see Fig. 5.

The control unit 114 comprises a computer system with functional modules or units, which are implemented in form of hardware, software or in form of a combination of both hardware and software. The computer system may comprise a microprocessor or the like and a computer program 115 in form of software, which can be loaded into the computer. Alternatively the computer program 115 is realized in form of a hardwired computer code. The computer program 115 comprises computer instructions in order to control the shimming coil 113 according to the invention. In particular the computer program 115 comprises computer instructions to control the amplitude and/or phase of the shimming current I_{S}. Alternatively the control unit may comprise an analogue control circuit for controlling the shimming coil 103. The analogue control circuit preferably comprises a transistor and/or an operating amplifier.

As illustrated in Fig. 6, generator coil 101 and sensor coil 108 are arranged on a common plane (XZ plane) and the axis 109 of the sensor coil 108 again is orientated perpendicular to the axis 110 of the generator coil 101 (normal alignment). Inside the sensor coil 108 the axis 109' of the sensor coil 108 is nearly perpendicular to the flux lines of the primary magnetic field 103. In the generator coil 101 an alternating current I_{G} is applied in order to generate a primary magnetic field 103. The primary magnetic field 103 induces an eddy current in the tissue 106 of the user's body and a secondary magnetic field is generated as a result of said eddy current (not shown in Fig. 6). In principle the primary and secondary magnetic fields exhibit the same shape as illustrated in Figs. 2 and 3.

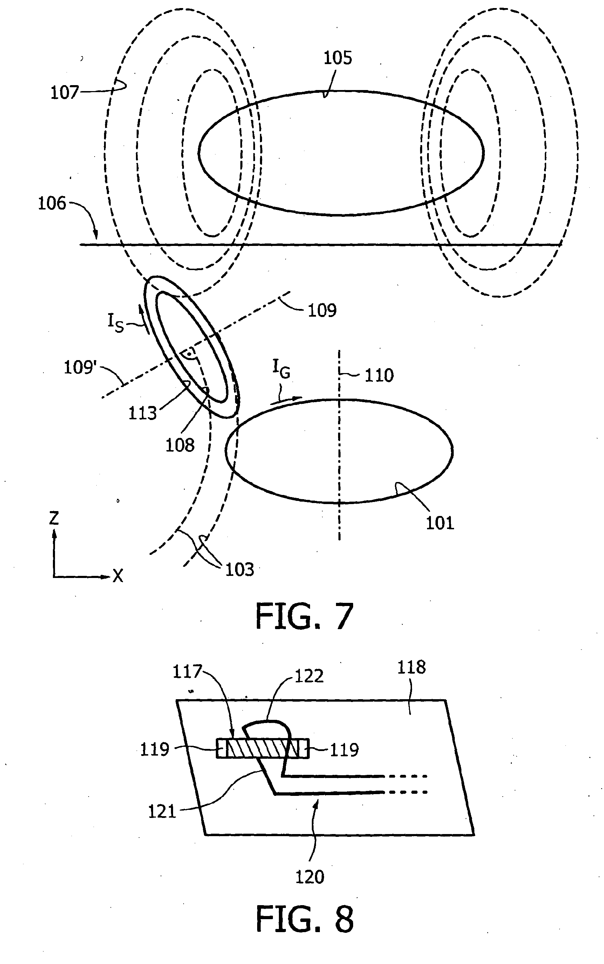

In Fig. 7 another embodiment of the system according to the invention is shown. In contrast to the very symmetric coil arrangement of Fig. 6, the sensor coil 108 and generator coil 101 are now positioned to each other in a non-symmetric way. More precisely, the sensor coil 108 (and the shimming coil 113 corresponding to the sensor coil 108) are rotated with respect to the generator coil 101. However, inside the sensor coil 108 the axis 109 of the sensor coil 108 is still substantially perpendicular to the flux lines of the primary magnetic field 103. Thus, using the shimming coil 113 (the axis 109' of the shimming coil being still parallel to the axis 109 of the sensor coil 108) the primary magnetic field 103 can be cancelled out in the sensor coil 108. As a result, in the sensor coil 108, only the secondary magnetic field 107 is sensed, said secondary magnetic field 107 being generated by the eddy currents 105 in the tissue 106 to be measured. In this embodiment the sensor coil 108 has not to be necessarily on the same plane as the generator coil 101. However, generator coil 101 and sensor coil 108 can be located on the same XZ plane.

The shimming coil 113 is implemented as an auxiliary winding of the sensor coil 108. In other words the shimming coil 113 is located around the sensor coil 108. The shimming coil 113 is arranged in a way that in the sensor coil 108 the primary magnetic field 103 is cancelled out, if the shimming current Is is set accordingly. For this purpose the shimming coil 113 and the sensor coil 108 are located on a common plane, with the axis 109' of the shimming coil 113 orientated parallel to the axis 109 of the sensor coil 108 for creating a magnetic flux in direction of the primary magnetic field 103.

The control unit 114 is adapted for providing a shimming current Is to the shimming coil 113. For this purpose the control unit 114 is connected to the generator coil 101 for receiving a partial amount of the current I_{G} of the generator coil 101. That partial amount (I_{S} = a I_{G} , wherein a is a gain coefficient) of the current I_{G} is then applied to the shimming coil 113. Thereby the amplitude of the current I_{S} to be supplied to the shimming coil 113 is automatically adjusted until the primary magnetic flux through the sensor coil 108 is zero. The adjustment is performed electronically and computer controlled using the control unit 114. For this purpose the control unit 114 measures the induced voltage in the sensor coil 108 and controls the amplitude of the shimming current Is until the induced voltage is zero. For optional controlling the phase of the shimming current Is the control unit 114 comprises a phase shifter module 116.

If the control unit 114 is implemented without the use of a computer software, the electronic adjustment may be performed automatically using a hardware based control unit or an analogue control circuit. The phase shifting mechanism can also be implemented in form of a hardware module.

Because the shimming coil 113 is considerably smaller than the generator coil 101 and the shimming current Is applied to the shimming coil 113 is very low compared to the generator coil current I_{G} applied to the generator coil 101 1 there are no eddy currents produced by the shimming coil 113.

A sensor coil adjustment as described above will preferably be carried out before each bio-impedance measurement, since between two measurements the measuring conditions might have been changed because of a temperature change or the like.

If tissue of a patient is to be measured, a setting to zero point can be performed. For this purpose the patient, e.g. laying on a bed, is asked to stop breathing and during this rest position the resulting measuring signal is regulated to zero by means of the shimming coil 113. As a result, field signals originating from eddy currents of the patient's rest position are suppressed.

In a preferred embodiment of the invention as illustrated in Fig. 8, the sensor coil is an SMD coil 117 attached to a printed circuit board 118 by means of two attachment points 119. The shimming coil 120 comprises a PCB-track 121 and a wire 122. The PCB-track 121 is positioned between said two attachment points 119 and runs beneath the SMD coil 117 and said wire 122 runs across the SMD coil 117. Alternatively a larger number of PCB-tracks 121 can be used. In this case the number of wires 122 has to be adapted accordingly.

It will be evident to those skilled in the art that the invention is not limited to the details of the foregoing illustrative embodiments, and that the present invention may be embodied in other specific forms without departing from the spirit or essential attributes thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. It will furthermore be evident that the word "comprising" does not exclude other elements or steps, that the words "a" or "an" do not exclude a plurality, and that a single element, such as a computer system or another unit may fulfil the functions of several means recited in the claims. Any reference signs in the claims shall not be construed as limiting the claim concerned.

### REFERENCE NUMERALS

- 1: generator coil
- 2: sensor coil
- 3: primary magnetic field
- 4: axis
- 5: eddy current
- 6: tissue
- 7: secondary magnetic field
- 8: sensor coil
- 9: axis sensor coil
- 10: axis generator coil
- 100: system
- 101: generator coil
- 102: (free)
- 103: primary magnetic field
- 104: (free)
- 105: eddy current
- 106: tissue
- 107: secondary magnetic field
- 108: sensor coil
- 109: axis sensor coil
- 110: axis generator coil
- 111: support
- 112: measuring unit
- 113: shimming coil
- 114: control unit
- 115: computer program
- 116: phase shifter module
- 117: SMD coil
- 118: printed circuit board
- 119: attachment point
- 120: shimming coil
- 121: PCB-track
- 122: wire

## Claims

1. A system (100) for inductively measuring the bio-impedance of a conductive tissue (106), the system (100) comprising
- a generator coil (101) adapted for generating a primary magnetic field (103), said primary magnetic field (103) inducing an eddy current in the conductive tissue (106), and
- a separate sensor coil (108; 117) adapted for sensing a secondary magnetic field, said secondary magnetic field being generated as a result of said eddy current, with the axis (109) of the sensor coil (108; 117) being orientated substantially perpendicular to the flux lines of the primary magnetic field (103),
**characterised in that** the system further comprises:
a shimming coil (113; 120) adapted for generating a tertiary magnetic field in a way that in the sensor coil (108; 117) the primary magnetic field (103) is cancelled out.

2. The system (100) as claimed in claim 1, **characterized in that** the shimming coil (113; 120) and the sensor coil (108; 117) are located in a way that the axis (109') of the shimming coil (113; 120) is orientated parallel to the axis (109) of the sensor coil (108; 117).

3. The system (100) as claimed in claim 1, **characterized in that** the shimming coil (113) is implemented as a number of auxiliary windings of the sensor coil (108).

4. The system (100) as claimed in claim 1, **characterized in that** a control unit (114) for controlling the shimming coil (113; 120) is connected to the shimming coil (113; 120), said control unit (114) being adapted for providing a shimming current (I_{S}) to the shimming coil (113; 120).

5. The system (100) as claimed in claim 4, **characterized in that** the control unit (114) is adapted for applying a partial amount (aI_{G}) of the generator coil current (I_{G}) to the shimming coil (113; 120).

6. The system (100) as claimed in claim 4, **characterized in that** the control unit (114) comprises a controllable potentiometer or a controllable resistor for adjusting the amplitude of the shimming current (Is).

7. The system (100) as claimed in claim 4, **characterized in that** the control unit (114) comprises a phase shifter module (116) adapted for shifting the phase of the shimming current (Is).

8. The system (100) as claimed in claim 1, **characterized in that** the shimming coil (113; 120) is considerably smaller than the generator coil (101) and/or the shimming current (I_{S}) applied to the shimming coil (113; 120) is very low compared to the generator coil current (I_{G}) applied to the generator coil (101).

9. The system (100) as claimed in claim 1, **characterized in that** the sensor coil (108; 117) is a surface mounted device coil (117) attached to a printed circuit board (118) by means of two attachment points (119) and the shimming coil (120) comprises a number of tracks (121) on the printed circuit board (118) and a corresponding numbers of wires (122), with said number of tracks (121) being positioned between said two attachment points (119) and beneath said surface mounted device coil (117) and said number of wires (122) running across said surface mounted device coil (117).

10. A method of inductively measuring the bio-impedance of a conductive tissue (106), the method comprising the steps of:
- arranging a generator coil (101) and a separate sensor coil (108; 117), with the axis (109) of the sensor coil (108, 117) being orientated substantially perpendicular to the flux lines of a primary magnetic field (103) generated by means of the generator coil (101), said primary magnetic field (103) inducing an eddy current in the conductive tissue (106), and
- sensing a secondary magnetic field by means of the sensor coil (108; 117), said secondary magnetic field being generated as a result of said eddy current,
**characterised in that** the method further comprises the step of:
- generating a tertiary magnetic field by means of a shimming coil (113; 120) in a way that in the sensor coil (108; 117) the primary magnetic field (103) is cancelled out.

11. A computer program (115) for operating a system (100) for inductively measuring the bio-impedance of a conductive tissue (106), the system (100) comprising a generator coil (101) adapted for generating a primary magnetic field (103), said primary magnetic field (103) inducing an eddy current in the conductive tissue (106), a separate sensor coil (108; 117) adapted for sensing a secondary magnetic field, said secondary magnetic field being generated as a result of said eddy current, with the axis (109) of the sensor coil (108; 117) being orientated substantially perpendicular to the flux lines of the primary magnetic field (103), and a shimming coil (113; 120), the program (115) comprising computer instructions to automatically controlling the shimming coil (113; 120) to generate a tertiary magnetic field in a way that in the sensor coil (108; 117) the primary magnetic fields (103) is cancelled out, when the computer program (115) is executed in a computer (114).

## Patentansprüche

1. System (100) zum induktiven Messen der Bioimpedanz eines leitfähigen Gewebes (106), wobei das System (100) Folgendes umfasst:
- eine Generatorspule (101) zum Erzeugen eines primären Magnetfelds (103), wobei das genannte primäre Magnetfeld (103) einen Wirbelstrom in das leitfähige Gewebe (106) induziert, und
- eine separate Sensorspule (108, 117) zum Messen eines sekundären Magnetfelds, wobei das genannte sekundäre Magnetfeld als Ergebnis des genannten Wirbelstroms erzeugt wird, wobei die Achse (109) der Sensorspule (108, 117) im Wesentlichen senkrecht zu den Flusslinien des primären Magnetfelds (103) ausgerichtet ist,
**dadurch gekennzeichnet, dass** das System weiterhin Folgendes umfasst:
- eine Shim-Spule (113, 120) zum Erzeugen eines tertiären Magnetfelds auf derartige Weise, dass das primäre Magnetfeld (103) in der Sensorspule (108, 117) aufgehoben wird.

2. System (100) nach Anspruch 1, **dadurch gekennzeichnet, dass** die Shim-Spule (113, 120) und die Sensorspule (108, 117) derartig angeordnet sind, dass die Achse (109') der Shim-Spule (113, 120) parallel zu der Achse (109) der Sensorspule (108, 117) ausgerichtet ist.

3. System nach Anspruch 1, **dadurch gekennzeichnet, dass** die Shim-Spule (113) als eine Anzahl von zusätzlichen Windungen der Sensorspule (108) implementiert ist.

4. System (100) nach Anspruch 1, **dadurch gekennzeichnet, dass** eine Steuereinheit (114) zum Steuern der Shim-Spule (113, 120) mit der Shim-Spule (113, 120) verbunden ist, wobei die Steuereinheit (114) dafür vorgesehen ist, einen Shim-Strom (I_{S}) an die Shim-Spule (113, 120) zu liefern.

5. System (100) nach Anspruch 4, **dadurch gekennzeichnet, dass** die Steuereinheit (114) dafür vorgesehen ist, eine Teilmenge (aI_{G}) des Generatorspulenstroms (I_{G}) an die Shim-Spule (113, 120) anzulegen.

6. System (100) nach Anspruch 4, **dadurch gekennzeichnet, dass** die Steuereinheit (114) ein regelbares Potentiometer oder einen regelbaren Widerstand zum Anpassen der Amplitude des Shim-Stroms (I_{S}) umfasst.

7. System (100) nach Anspruch 4, **dadurch gekennzeichnet, dass** die Steuereinheit (114) ein Phasenverschiebungsmodul (116) umfasst, das vorgesehen ist, um die Phase des Shim-Stroms (I_{S}) zu verschieben.

8. System (100) nach Anspruch 1, **dadurch gekennzeichnet, dass** die Shim-Spule (113, 120) wesentlich kleiner ist als die Generatorspule (101) und/oder der der Shim-Spule (113, 120) zugeführte Shim-Strom (I_{S}) sehr niedrig im Vergleich zu dem Generatorspulenstrom (I_{G}) ist, der der Generatorspule (101) zugeführt wird.

9. System (100) nach Anspruch 1, **dadurch gekennzeichnet, dass** die Sensorspule (108, 117) eine oberflächenmontierte Spule (117) ist, die mittels zwei Befestigungspunkten (119) auf einer Leiterplatte (118) montiert ist, und die Shim-Spule (120) eine Anzahl von Leiterbahnen (121) auf der Leiterplatte (118) und eine entsprechende Anzahl von Drähten (122) umfasst, wobei die genannte Anzahl von Leiterbahnen (121) zwischen den genannten beiden Befestigungspunkten (119) und unterhalb der genannten oberflächenmontierten Spule (117) angeordnet ist und die genannte Anzahl von Drähten (122) quer über die genannte oberflächenmontierte Spule (117) verläuft.

10. Verfahren zum induktiven Messen der Bioimpedanz eines leitfähigen Gewebes (106), wobei das Verfahren die folgenden Schritte umfasst:
- Anordnen einer Generatorspule (101) und einer separaten Sensorspule (108, 117), wobei die Achse (109) der Sensorspule (108, 117) im Wesentlichen senkrecht zu den Flusslinien eines mittels der Generatorspule (101) erzeugten primären Magnetfelds (103) ausgerichtet ist, wobei das genannte primäre Magnetfeld (103) einen Wirbelstrom in das leitfähige Gewebe (106) induziert, und
- Messen eines sekundären Magnetfelds mit Hilfe der Sensorspule (108, 117), wobei das genannte sekundäre Magnetfeld als Ergebnis des genannten Wirbelstroms erzeugt wird,
**dadurch gekennzeichnet, dass** das Verfahren weiterhin folgenden Schritt umfasst:
- Erzeugen eines tertiären Magnetfelds mit Hilfe einer Shim-Spule (113, 120) auf derartige Weise, dass das primäre Magnetfeld (103) in der Sensorspule (108, 117) aufgehoben wird.

11. Computerprogramm (115) zum Betreiben eines Systems (100) zum induktiven Messen der Bioimpedanz eines leitfähigen Gewebes (106), wobei das System (100) Folgendes umfasst: eine Generatorspule (101) zum Erzeugen eines primären Magnetfelds (103), wobei das genannte primäre Magnetfeld (103) einen Wirbelstrom in das leitfähige Gewebe (106) induziert, eine separate Sensorspule (108, 117) zum Messen eines sekundären Magnetfelds, wobei das genannte sekundäre Magnetfeld als Ergebnis des genannten Wirbelstroms erzeugt wird, wobei die Achse (109) der Sensorspule (108, 117) im Wesentlichen senkrecht zu den Flusslinien des primären Magnetfelds (103) ausgerichtet ist, und eine Shim-Spule (113, 120); wobei das Programm (115) Computeranweisungen umfasst, um die Shim-Spule (113, 120) automatisch so zu steuern, dass ein tertiäres Magnetfeld auf derartige Weise erzeugt wird, dass das primäre Magnetfeld (103) in der Sensorspule (108, 117) aufgehoben wird, wenn das Computerprogramm (115) in einem Computer (114) ausgeführt wird.

## Revendications

1. Système (100) pour la mesure inductive de la bio-impédance d'un tissu conducteur (106), le système (100) comprenant :
- une bobine génératrice (101) qui est adaptée de manière à générer un champ magnétique primaire (103), ledit champ magnétique primaire (103) induisant un courant de Foucault dans le tissu conducteur (106), et
- une bobine de capteur séparée (108 ; 117) qui est adaptée de manière à détecter un champ magnétique secondaire, ledit champ magnétique secondaire étant généré en conséquence dudit courant de Foucault, l'axe (109) de la bobine de capteur (108 ; 117) étant orientée d'une manière sensiblement perpendiculaire aux lignes de flux du champ magnétique primaire (103),
**caractérisé en ce que** le système comprend encore :
- une bobine de calage (113 ; 120) qui est adaptée de manière à générer un champ magnétique tertiaire de telle façon que dans la bobine de capteur (108 ; 117) le champ magnétique primaire (103) soit annulé.

2. Système (100) selon la revendication 1, **caractérisé en ce que** la bobine de calage (113 ; 120) et la bobine de capteur (108 ; 117) se situent de telle façon que l'axe (109') de la bobine de calage (113 ; 120) soit orienté en parallèle à l'axe (109) de la bobine de capteur (109; 117).

3. Système (100) selon la revendication 1, **caractérisé en ce que** la bobine de calage (113) est mise en oeuvre en tant qu'un nombre d'enroulements auxiliaires de la bobine de capteur (108).

4. Système (100) selon la revendication 1, **caractérisé en ce qu'**une unité de commande (114) pour commander la bobine de calage (113 ; 120) est connectée à la bobine de calage (113 ; 120), ladite unité de commande (114) étant adaptée de manière à fournir un courant de calage (I_{S}) à la bobine de calage (113 ; 120).

5. Système (100) selon la revendication 4, **caractérisé en ce que** l'unité de commande (114) est adaptée de manière à appliquer une quantité partielle (aI_{G}) du courant de bobine génératrice (I_{G}) à la bobine de calage (113 ; 120).

6. Système (100) selon la revendication 4, **caractérisé en ce que** l'unité de commande (114) comprend un potentiomètre contrôlable ou une résistance contrôlable pour ajuster l'amplitude du courant de calage (I_{S}).

7. Système (100) selon la revendication 4, **caractérisé en ce que** l'unité de commande (114) comprend un module déphaseur (116) qui est adapté de manière à décaler la phase du courant de calage (I_{S}).

8. Système (100) selon la revendication 1, **caractérisé en ce que** la bobine de calage (113 ; 120) est considérablement plus petite que la bobine génératrice (101) et/ou **en ce que** le courant de calage (I_{S}) qui est appliqué à la bobine de calage (113 ; 120) est très bas par rapport au courant de bobine génératrice (I_{G}) qui est appliqué à la bobine génératrice (101).

9. Système (100) selon la revendication 1, **caractérisé en ce que** la bobine de capteur (108 ; 117) est une bobine de dispositif montée en surface (117) qui est fixée à une carte de circuit imprimé (118) au moyen de deux points de fixation (119) et **en ce que** la bobine de calage (120) comprend un certain nombre de pistes (121) sur la carte de circuit imprimé (118) et un nombre correspondant de fils (122), ledit nombre de pistes (121) étant positionnées entre lesdits deux points de fixation (119) et au-dessous de ladite bobine de dispositif montée en surface (117) et ledit nombre de fils (122) s'étendant à travers ladite bobine de dispositif montée en surface (117).

10. Procédé pour la mesure inductive de la bio-impédance d'un tissu conducteur (106), le procédé comprenant les étapes suivantes consistant à :
- arranger une bobine génératrice (101) et une bobine de capteur séparée (108 ; 117), l'axe (109) de la bobine de capteur (108 ; 117) étant orienté d'une manière sensiblement perpendiculaire aux lignes de flux d'un champ magnétique primaire (103) qui est généré au moyen de la bobine génératrice (101), ledit champ magnétique primaire (103) induisant un courant de Foucault dans le tissu conducteur (106), et
- détecter un champ magnétique secondaire au moyen de la bobine de capteur (108 ; 117), ledit champ magnétique secondaire étant généré en conséquence dudit courant de Foucault,
**caractérisé en ce que** le procédé comprend encore l'étape suivante consistant à :
- générer un champ magnétique tertiaire au moyen d'une bobine de calage (113 ; 120) de telle façon que dans la bobine de capteur (108 ; 117) le champ magnétique primaire (103) soit annulé.

11. Programme informatique (115) d'exploitation d'un système (100) pour la mesure inductive de la bio-impédance d'un tissu conducteur (106), le système (100) comprenant une bobine génératrice (101) qui est adaptée de manière à générer un champ magnétique primaire (103), ledit champ magnétique primaire (103) induisant un courant de Foucault dans le tissu conducteur (106), une bobine de capteur séparée (108 ; 117) qui est adaptée de manière à détecter un champ magnétique secondaire, ledit champ magnétique secondaire étant généré en conséquence dudit courant de Foucault, l'axe (109) de la bobine de capteur (108 ; 117) étant orienté d'une manière sensiblement perpendiculaire aux lignes de flux du champ magnétique primaire (103), et une bobine de calage (113 ; 120), le programme (115) comprenant des instructions informatiques pour commander automatiquement la bobine de calage (113 ; 120) afin de générer un champ magnétique tertiaire de telle façon que dans la bobine de capteur (108 ; 117) le champ magnétique primaire (103) soit annulé lorsque le programme informatique (115) est exécuté dans un ordinateur (114).
